# EUROPEAN PATENT SPECIFICATION

(11) **EP 4 558 231 B1**
(45) Date of publication and mention of the grant of the patent: **01.07.2026**
(21) Application number: 23750680.3
(22) Date of filing: 19.07.2023
(51) Int. Cl.: A63B 69/00, A61B 5/16, A61M 21/00, A61N 5/06, A63B 43/06, A63B 71/04, A63B 71/06, A63B 71/14, H05B 47/16, A63B 63/00, A63B 102/16

(54) **TRAINING SYSTEM AND METHOD OF USE THEREOF**
TRAININGSSYSTEM UND VERFAHREN ZUR VERWENDUNG DAVON
SYSTÈME D'ENTRAÎNEMENT ET SON PROCÉDÉ D'UTILISATION

(30) Priority: 20.07.2022 GB 202210588
(43) Date of publication of application: 28.05.2025
(73) Proprietor: OKKULO LIMITED, Newcastle upon Tyne Tyne and Wear NE1 4BF (GB)
(72) Inventor: O'CONNOR, Mel, Newcastle upon Tyne Tyne and Wear NE1 4BF (GB)
(74) Representative: Tomkinson, Alexandra
(86) International application number: PCT/GB2023/051895
(87) International publication number: WO 2024/018203

(56) References cited:
- US-A- 5 709 645
- US-A1- 2013 266 918
- US-A1- 2016 367 856

## Description

This invention relates to a training system and to a method of use thereof.

Although the following description refers almost exclusively to a sports training system, it will be appreciated by persons skilled in the art that the training system of the present invention could be used to train one or more individuals to improve their visualisation and/or ability to locate an object in lower light conditions and/or for learning and enhancing a user's responses and visuomotor skills for use in any application, such as for example a military application, a security application, a non-sporting application, therapeutic applications and/or the like.

There are many pursuits or applications in which an improvement or speeding up of a user's hand-eye or foot-eye coordination or reaction time could improve the performance of the user involved in said pursuits or applications. For example, in ball sports such as cricket, an improvement in a player's hand-eye coordination could improve their ability to play, hit, catch or stop a ball and to do so at higher ball speeds. In tennis, an improvement in the reaction time of a tennis player could improve the percentage of serves they are able to return in a game and also to do so at higher ball speeds. There are therefore clear advantages for providing a system that would allow a user to train, improve and speed up their reaction times and/or hand-eye or foot-eye coordination, and thus their ability to interact successfully with a moving sports object (which because of unavoidable neural processing delays always requires the user to predict where the ball or sporting object will be before they actually see it there).

An example of such a training system is disclosed in the Applicant's co-pending patent application WO2019/092431, that is directed to an apparatus and method for allowing a user to learn and enhance visuomotor skills. The apparatus includes an enclosed space or housing to provide a near zero background level of visible illumination, yet still provide visible luminous training elements within the space, that together allow a training environment to be provided in which a user's sensory system is forced to work in conditions under which the speed of visual processing is reduced. The luminous training elements are produced by the fluorescence of coatings on the training elements illuminated by ultraviolet (UV) light, or self-emitting sources of illumination provided in the training elements. The background illumination is provided by UV light sources that emit non-visible light having a fixed wavelength of between 300-400 nm that allows the training elements to fluoresce. The system is arranged such that the luminance level of the luminous training elements is greater than the luminance level of the background level of illumination within the training area.

Scotopic luminance levels are those at which only the rods, the most sensitive photoreceptors, are stimulated. Photopic luminance levels are those at which only the cones are stimulated. Mesopic luminance levels are those intermediate levels at which both the cones and rods are stimulated simultaneously. Mesopic vision begins at luminance levels of approximately 0.01cd/m². The luminance level at which it ends depends to a large extent on the spectral composition of the stimuli being viewed, their sizes and locations of the retina. However, the rods are thought to be fully saturated a luminance levels of about 2000 scotopic trolands or about 300 scotopic cd/m². Rod vision is much slower than cone vision, but cone vision also slows down substantially as the light level is decreased from high photopic to low mesopic levels. The Applicant's found that their previous training system was optimal where the rods were not being stimulated and the light levels of the cones was low, thus making cone vision slow. They found the optimal ambient background level of illumination to be in the range of 0.001 cd/m² to 200 cd/m². The Applicant's also found that the optimal luminous of the training elements to be above approximately 0.001 cd/m² or higher.

There are two main theories as to how the Applicant's previous apparatus is able to bring about an improvement in a user's visuomotor skills: a first theory termed "adaptive recalibration and plasticity", and a second theory termed "visual attentiveness".

In adaptive recalibration and plasticity, as a result of a user training for a particular activity in the dark or at low light levels, the user is provided with the opportunity to rehearse a sequence of visuomotor actions required to perform a particular task under conditions in which the speed of visual processing is slowed down by the natural process of light adaptation (which are known to speed up visual processing in the light and slow it down in the dark). Thus, the user has less time to respond to a particular task and, in order to perform the task successfully, the user's visuomotor responses to the stimuli must be performed more quickly to advance their response. The user's visuomotor performance is thus sped up, effectively recalibrating visual processing to temporally align with the slower processing of visual information at the low light level. When the user reverts to normal bright light conditions and faster speeds of processing, rather than performing worse because the response is now too early, the faster visuomotor responses translate into performance enhancements and an ability to respond better and faster to the task not only at normal ball speeds but also at increased speeds.

In visual attentiveness, as a result of a user training for a particular activity in the dark, this forces the user to concentrate and focus and to exclude distracting information. This learnt behaviour can also bring about an improvement in performance of the user when in normal bright light conditions.

The Applicant has continued to develop their training system and understanding of how visual performance is enhanced. Document US2016/367856 A1 describes a boxing-based fitness training regimen which takes place in a training area suffused with UV light from one or more UV light sources. Non-UV lighting in the training area is adjusted to a level of semi-darkness, in which non-luminescent objects are visible but luminescent objects clearly stand out.

It is therefore an aim of the present invention to provide an improved training system.

It is a further aim of the present invention to provide an improved method of using a training system.

It is a yet further aim of the present invention to provide an improved sports training system and/or method of use thereof.

It is a yet further aim of the present invention to provide a therapeutic training system and/or method of use thereof.

According to a first aspect of the present invention there is provided a training system according to claim 1.

By providing the training system of the present invention with means for adjusting the background illumination and/or physical element illumination, together or independently, this allows adjustment of the training emphasis, visuomotor skills training and/or therapeutic effect provided by the system. Thus, for example, once the background illumination means and/or physical element illumination means are in an "on" or operational condition, the level of illumination provided by the same can be adjusted. This contrasts with prior art training systems wherein the illumination provided by the background illumination means and/or physical element illumination means is fixed in use of the systems. As such, the training system of the present invention provides a much wider range of applications of use, allows significantly improved fine tuning of visuomotor skills training, and provides a system that can be personalised for a particular user or group of users.

Preferably the background illumination provided by the background illumination means and/or the physical element illumination provided by the physical element illumination means includes, consists or comprises visible light (i.e., light visible to a human eye).

Thus preferably, in one embodiment, the adjustment means allows adjustment of visible light of the background illumination and/or the physical element illumination.

In one embodiment the adjustment means allows adjustment of one or more parameters and/or conditions relating to the background illumination, the background illumination means, the physical element illumination and/or the physical element illumination means, such as for example, adjustment of any or any combination of: the wavelength composition of the light emitted from the illumination means (and thus its colour), the brightness of the light emitted from the illumination means, the intensity of the light emitted from the illumination means, the type of light emitted from the illumination means, the ratio of two or more different lights of different wavelengths emitted by the illumination means, the contrast between the light emitting by the background illumination means and the physical element illumination means and/or the like.

It is to be noted that reference to "illumination means" per se herein can include the background illumination means and/or the physical element illumination means.

Preferably the adjustment means can be used to adjust two or more of the parameters and/or conditions relating to the illumination and/or illumination means simultaneously and/or independently of each other. For example, a user could adjust the wavelength of light emitted from the illumination means independently of the brightness of the light emitted from the illumination means. In a further example, a user could adjust the brightness of two or more different lights emitted by the system simultaneously.

Preferably the difficulty of the training task being performed by a user of the system can be made easier or more difficult by changing the contrast between the physical element illumination and the background illumination in use.

Preferably the adjustment means are arranged to allow adjustment of the background illumination means and/or training element illumination means so as to result in an adjustment in the stimulation of one or more cones and/or rods of a human eye of the person using the system in use.

Preferably the adjustment means are arranged to allow adjustment of the background illumination means and/or training element illumination means so as to result in stimulation of a user's vision in the mesopic, photopic and/or scotopic range or ranges of vision.

In one embodiment, adjustment of illumination in the system via the adjustment means takes place manually, or automatically as a result of one or more predetermined conditions being met. For example, the system could be arranged such that once a user has achieved a pre-determined number of training tasks successfully, and/or after a pre-determined period of time, the illumination of the system is automatically changed.

In one embodiment the pre-determined conditions can be determined or are selectable by a user of the system, the manufacturer of the system and/or the like.

In one embodiment the adjustment means includes one or more user actuation means or devices, such as for example any or any combination of one or more dials, buttons, knobs, switches, keyboards, joysticks, touch screen display and/or the like.

Preferably the adjustment means and/or the one or more user actuation means include any device or means that allow adjustment and/or actuation of the illumination or illumination means in use.

In one example, the system includes a display screen, such as for example a touch screen display, wherein one or more pre-determined background illumination options and/or training element illumination options can be provided and/or selected by a user of the system.

In one example, the system of the present invention includes a control device or unit and the adjustment means are provided on and/or associated with the control device or unit.

Preferably the adjustment means, the user actuation means and/or the control device or unit can be located within a pre-determined training area relating to the system or can be located remote from a pre-determined training area.

Preferably the control device or unit includes micro-processing means, a computer, a tablet, a housing, a smart phone, a server and/or the like.

Preferably the control device or unit includes hardware and/or software for carrying out one or more processes; memory means for storing data relating to the system and/or training tasks being performed; communication means for communicating data with and/or between one or more electronic devices and/or the like.

In one embodiment the communication means can include wired or wireless means, Bluetooth, WiFi, Internet, Infra-red (IR), one or more radio frequency signals and/or the like.

In one embodiment the user actuation means are provided on and/or associated with the control device or unit.

For example, four buttons or dials can be provided on a control unit housing; a first button for controlling UV light, a second button for controlling blue light, a third button for controlling green light, a fourth button for controlling red light.

In one embodiment, the adjustment means for adjusting the brightness of the illumination means can be used to change the brightness of a single light source or single coloured light source independently and/or separately to any other light source of the system. Alternatively, the adjustment means for adjusting the brightness of the illumination means can adjust two or more different light sources of the system simultaneously. This can allow, for example, the ratio of the two or more different light sources to be maintained but to allow adjustment of the brightness of the light sources.

In one example, the type of light emitted from the illumination means can be polarised light, non-polarised light, partially polarised light, different states of polarised light and/or the like.

In one example the light emitted from the illumination means is a continuous or substantially continuous light, a non-continuous or substantially non-continuous light, a pulsed light; a pre-determined or random pattern, sequence or sequences of light(s) and/or the like.

Preferably the light emitted from the illumination means is visible light.

Preferably the illumination means includes any or any combination of any one or more devices that are able to generate and emit light therefrom in use, one or more lights, light emitting diodes (LEDs), one or more halogen light sources, one or more high intensity discharge (HID) light sources and/or the like.

In a preferred embodiment the illumination means includes one or more LEDs so as to provide an energy efficiency light source with good and accurate light of pre-determined wavelength.

In one example the illumination means includes one or more arrays of LEDs.

Preferably a plurality of illumination means are provided and each illumination means is arranged a spaced distance apart from another illumination means within the training area, and further preferably are arranged an equidistance apart within the training area and/or an enclosed area.

In one example the illumination means are provided in a housing wherein an opening in the housing from which illumination is emitted in use has protection means provided on and/or associated with the same to protect the illumination means from damage, environmental conditions, water, ingress of dirt and/or the like. For example, the protection means could include a cover, a mesh cover, a tapered opening and/or the like. This helps to prevent the physical element, if directed near to the illumination means, from breaking and/or damaging the same in use. This is particularly the case if the physical element is a ball for example.

In one embodiment the background illumination means and/or the physical element illumination means are arranged so as to emit light having two or more different wavelengths and/or having two or more different colours.

For example, the background illumination means could include a first illumination means for emitting a first colour and/or wavelength or light or red visible light; and a second illumination means for emitting a second colour and/or wavelength of light or blue visible light.

In a further example, the background illumination means could further include third illumination means for emitting a third colour and/or wavelength of light or green visible light.

Preferably the first, second and/or at least third light colours and/or wavelengths are different.

It will be appreciated that any combination of different coloured and/or wavelengths of light could be provided by the background illumination means and/or the physical element illumination means as required.

Preferably the adjustment means allows the ratio of the two or more different coloured and/or wavelengths of lights to be adjusted in use.

In one example, the two or more different coloured and/or wavelengths of light are within the visible spectrum.

In one example, at least one of the illumination means provides white light or a combination of different coloured lights and/or wavelengths of light that when mixed or added together produce white light.

The illumination provided outside of the visible spectrum is used to make the physical element fluoresce in use.

In embodiment the background illumination means includes at least one red LED, at least one green LED and/or at least one blue LED.

Preferably when two or more different coloured and/or wavelengths of light are used in the system, they can be added together to create a further coloured light, can create white light and/or the like.

In one embodiment three of more different illumination means are provided to provide light of three or more different colours and/or wavelengths and/or to create white light.

In one embodiment four or more different illumination means are provided to provide light of four or more different colours and/or wavelengths and/or to create white light. For example, the four or more different illumination means could include a blue light source, a red light source, a green light source and a UV light source.

In one embodiment the illumination means includes three or more light sources arranged to emit light of three or more different colours such that, when the emitted light is mixed or added together in one or more intensity ratios they can produce a large range of possible colours, such as for example, reds, oranges, yellows, greens, blues, violets and/or mixtures thereof.

In one embodiment the blue light emitted by the illumination means is centered on a wavelength of approximately 450nm +/- 10nm.

In one embodiment the red light emitted by the illumination means is centered on a wavelength of approximately 630nm +/-10nm.

In one embodiment the green light emitted by the illumination means is centered on a wavelength of approximately 530nm +/-10nm.

In one embodiment the illumination means provides white light, preferably white light made up of a mixture of spectral components between 400-700nm wavelength, and further preferably white light made up of spectral components between 420nm-700nm.

In one embodiment filter means are provided on and/or associated with the illumination means to provide illumination of one or more colours, specific wavelengths, patterns and/or the like.

In one embodiment one of the filter means or illumination means is movable relative to the other of the filter means or illumination means to allow adjustment of the luminance provided by the illumination means in use.

In one embodiment the filter means is fixed relative to the illumination means.

In one embodiment the background illumination means includes UVA light.

Preferably the UV light used in the system is approximately 365nm +/-10nm.

In one embodiment the background illumination provided by the background illumination means is adjustable from 0 or 0.0001 to 1000 cd/m².

Preferably the background illumination means and/or the training element illumination means can be moved between an "on" or operational condition and an "off" or non-operational conditions. Further preferably the adjustment of the background illumination means and/or the training element illumination means takes place when the illumination means is in the on or operational condition.

In one embodiment the background illumination provided by the background illumination is adjustable from zero up to full daylight lighting levels. As such, a user of the system can perform the training task in relatively low lighting conditions or relatively bright lighting conditions and/or somewhere between the two condition extremes.

In one embodiment the illumination provided by the physical element illumination means is adjustable to a level equal to or greater than 0.001cd/m².

Preferably the physical element illumination means is adjustable to a level such that the illumination associated with the same is bright enough to be seen by a user's cones and/or above the mesopic vision limit.

In one embodiment the physical element illumination means are locatable directly in or on the physical element and light or luminance is emitted from the same in use.

For example, the physical element illumination means could be a self-emitting illumination source, such as an LED light source located in the physical element.

The physical element illumination means are provided remotely from the physical element but allow light or luminance to be emitted from the physical element or to illuminate the physical element. The physical element illumination means includes a coating provided on and/or associated which the physical element which emits visible light under certain conditions and the coating is a UV sensitive coating. In one example the physical element illumination means could include a remote light source or light beam that is directed onto and/or tracks the physical element to illuminate the same in use.

The background illumination means provides illumination that can stimulate and/or activate the physical element illumination means in use. The background illumination means includes a UV light source and the physical element illumination means includes a UV sensitive coating that emits visible light when the UV light source is directed onto the same in use.

In one example, the background illumination means and at least part of the physical element illumination means are the same light source.

In one embodiment the background illumination means and/or physical element illumination means are adjustable to one of a number of possible user selectable arbitrary positions.

In one embodiment the background illumination means/or physical element illumination means are adjustable to one of a number of possible pre-determined positions.

In one embodiment the system includes a training area provided in an enclosed space, such as for example a housing, room, compartment, tent, enclosure and/or the like.

Preferably the training area is pre-defined and/or a definite size and/or shape.

In one embodiment the system includes a training area and preferably said training area is not an enclosed space.

In one embodiment a radiation absorbing coating and/or layer is provided on and/or associated with an interior surface of the enclosed training area, space or housing.

Preferably the background illumination means are arranged to provide at least a degree of illumination within the training area. For example, the background illumination can be provided in the training area or can be provided external to the training area but arranged so as to provide illumination within the training area.

In one embodiment, use of the term "background illumination" is defined as lighting provided within an area or locality in which the system is used to provide general lighting within the area or locality.

In one embodiment, user of the term "physical element illumination" is defined as lighting provided specifically to illuminate a physical element, such as for example, a physical element being used within an area or locality in which the system is being used.

Preferably the physical element background illumination means are locatable in the training area so that a user can carry out a task relating to the physical element within the training area.

In one embodiment the system includes an item of eyewear and/or head wear for wearing by a user.

Preferably the eyewear and/or headwear are arranged to be worn by a user to provide an enclosed or substantially enclosed space area around the user's eye or eyes.

Preferably the background illumination means are arranged to provide illumination or the option of illumination within the enclosed or substantially enclosed space area around the user's eyes, or to the space surrounding the user's eye or eyes. As such, rather than the entire training area being illuminated by the background illumination means, only the user's field of view or immediate field of view is provided with background illumination from the background illumination means.

For example, the eyewear and/or headwear could include a pair of glasses, goggles, one or more eyepieces, one or more lenses, a virtual reality (VR) headset, a headset and/or the like.

Preferably when the eyewear and/or headwear includes a VR headset, real world conditions, one or more training environments and/or the like can be imposed on the visual display of the VR headset, thereby making the task being performed by a user when wearing the VR headset more realistic and/or could be used to introduce additional stimuli or challenges into the system.

In one embodiment the system includes sensing means or one or more sensing devices for sensing the position, speed and/or movement of one or more of the physical elements in use.

Preferably the sensing means are provided on, in and/or associated with eyewear, headwear, a training area, a predetermined surface within the training area, with the physical element and/or the like.

In one embodiment the sensing means can be arranged to allow sensing, measuring and/or recording of data relating to the one or more training tasks and/or the physical element in use.

In one embodiment the sensing means includes one or more sensors, training element sensors, accelerometers, GPS sensors, movement sensors and/or the like.

In one embodiment the background illumination level is arranged to be relatively lower than the illumination level of the physical element in use.

Preferably the training task is a sport task or game and the physical element is an element of the sport or game. For example, the training tasks could be to kick a ball, hit a ball, save or catch a ball and/or the like.

Preferably the training task is an everyday task that a user may be required to perform in everyday life and the physical element is an object used to perform the everyday task.

Preferably the physical element can be a ball, a bat, a club, a golf club, a glove, a racquet, a goal, one or more markings for a training area or goal and/or the like.

According to a further aspect of the present invention there is provided a method of using a training system according to claim 15.

Preferably the method includes the steps of performing an initial amount of training of the training task according to a pre-determined training regime and using the system of the present invention.

According to further embodiments of the present invention there is provided a sports training system and/or method of use thereof; a military training system and/or method of use thereof; a security training system and/or method of use thereof; a training system providing therapeutic benefit and/or method of using the same.

According to one embodiment of the present invention there is provided a training system, said training system including:
- background illumination means to provide background illumination for a user's vision in use;
- at least one physical element relating to a training task to be performed by the user in use;
- wherein physical element illumination means are provided on, in and/or associated with the at least one physical element to allow the physical element to be illuminated in use;
the background illumination means and the physical element illumination means are each arranged to provide visible illumination.

Preferably the background illumination means is arranged to provide light of at least two different colours, of at least two different wavelengths and/or of at least two different brightnesses.

Thus, the present invention in some embodiments can provide:
a) An enclosed sports training arena within which the intensity and spectral composition of the illumination can be controlled. In one example, four different light sources of different visible wavelengths can be provided.
b) A lighting system wherein at least one of the light sources is UVA light that is largely invisible to the human eye but that can be varied in intensity to cause a coated physical object, such as a football, to fluoresce with visible light so that it can be seen by a player in low light conditions.
c) A lighting system wherein three of four light sources are set to different visible wavelengths of light and are adjustable to be set to one of a number of different intensity ratios to generate a white illumination of the background illumination. In one example, the three light sources are centered on blue light (450nm), green light (530nm) and red light (630nm). In one example, the white light created by the different light sources is adjustable from zero or darkness to 1000 cd/m².
d) A lighting system wherein the relative intensities of light sources of different visible wavelengths can be adjusted to vary the stimulation of one or more rods and/or three cones of a human eye, and particularly preferably to allow variation in stimulation of the three different cones and rods in the human eye.
e) A lighting system and/or sports training area wherein the background lighting and thus the state of adaptation of a user's visual system can be varied from near darkness (i.e. 0.0001 cd/m²) to bright indoor lighting levels (i.e. 1000 cd/m²).
f) A lighting system and/or sports training area within which the adaptation and speed of visual processing can be controlled by changing the illumination within the same.
g) A training area of variable size that can be optimized to allow specific training tasks associated with a different application and/or sport to be performed, such as for example baseball pitching and batting , cricket bowling and batting, boxing, football shooting and goal keeping, table tennis, tennis and/of the like.

Embodiments of the present invention will now be described with reference to the following figures, wherein:
Figure 1 is an example of a training system for use in an enclosed training area according to one embodiment of the present invention;
Figure 2 is an example of a training system according to a further embodiment of the present invention when the training system includes an item of headwear and/or eyewear in the form of a VR headset in which at least the background illumination means are provided;
Figure 3 is a graph showing the scotopic (black curve) and photopic (white curve) luminous efficacy functions of a human eye in lumens per W (lm/W).

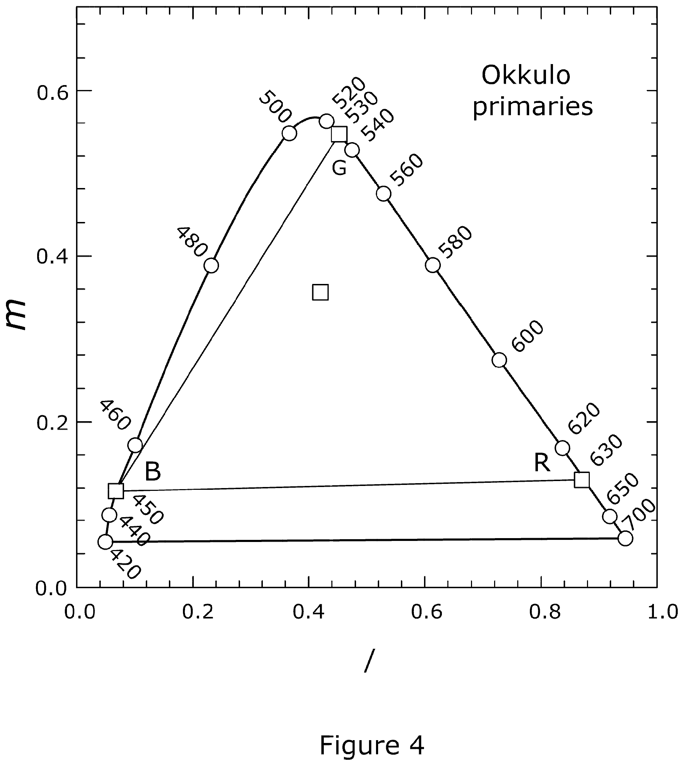

Figure 4 shows a chromaticity diagram. The axes of the graph relate to the cone excitation of a human eye produced by lights of different wavelengths, wherein s(λ) = 1-[1(λ) + m(λ)], so that when l(λ) and m(λ) are small, as in the bottom left hand of the diagram, s(λ) is large. The black line, known as the "spectrum locus" shows the colours of spectral lights (single wavelength or "monochromatic" colours) that make up the spectrum seen in a rainbow. The line joining the two ends of the locus is called the purple line. All physically realisable colours lie within the area delimited by the spectrum locus and the purple line. All physically realisable colours of the present invention, in one embodiment, lie within the white triangle joining the red, green, blue primaries shown by the red, green and blue squares.

The training system of the present invention is arranged to utilise the natural functions of a user's eye or eyes and brain to generate a beneficial training and/or therapeutic effect for the user. The human visual system will slow down processing of eye and brain signals in the dark or in relatively low conditions and will speed up the processing of eye and brain signals in light or in relatively bright light conditions. The training system of the present invention provides an adjustable system that can alter the background level of lighting or luminance in a locality to which a user's eye or eye is exposed and/or can alter the lighting or luminance of a physical element within the locality to which a user is required to perform one or more tasks with, to achieve a range of different visuomotor responses and/or therapeutic responses in the user. In particular, it has been found that by training a user to perform one or more tasks relating to a physical element in relatively low level lighting conditions, the user performs the one or more training tasks relating to the physical element significantly better when the lighting conditions are returned to a brighter level or normal lighting levels (i.e. normal daylight conditions). Furthermore, it has been found that by training a user to perform one or more training tasks relating to a physical element in relatively low level lighting conditions, the user enjoys an improved level of concentration, focus and emotional wellbeing when the user returns to normal lighting levels or relatively bright levels of light. The present invention therefore has a surprising and unexpected therapeutic effect.

Human vision has three types of daytime light-sensitive photoreceptors called Long (L), Middle (M) and Short (S) wavelength sensitive cones. Each type of cone is most sensitive to a different part of the visible spectrum. For example, the S-cones are most sensitive to violet and blue visible light, the M-cones are most sensitive to green visible light and the L-cones are most sensitive to green-yellow visible light. There is also another type of light sensitive photoreceptor in the human eye called rods, which are generally more light sensitive than cones, are most sensitive in the green-blue part of the spectrum and are optimized for night-time vision. In particular, cones are more sensitive in the green/blue part of the visible spectrum. Therefore, in one embodiment the present invention allows the background level of lighting or luminance and/or the lighting or luminance of the physical element to be adjusted in such a way so as to stimulate a pre-determined, and optionally user selectable, particular type or combination of types of photoreceptor within a user's eye. This allows a user to control very specifically the visuomotor skill that is to be trained and/or the therapeutic effect that is desired. This has not previously been possible with prior art training systems.

The processing time taken between an image being formed on a user's retina and the user's reaction time to something unanticipated in the image takes a substantial amount of time. Traditional estimates of how fast a user can respond to an unexpected visual event range from about 200-300ms. The user's reaction time to a visual event changes according to changes in lighting level, such as background lighting levels and/or the luminance of a physical element. In relatively low lighting conditions, a user's natural response time to an event is slower than for the same event in relatively brighter lighting conditions. As such, by allowing a user to train for a particular task at relatively low lighting levels, the user learns to speed up their visual processing and hence improve their reaction time to perform the task at these low lighting levels. When the user then comes to perform the same task at relatively brighter lighting levels, their reaction time is significantly improved beyond their pre-trained reaction times at the same higher lighting levels.

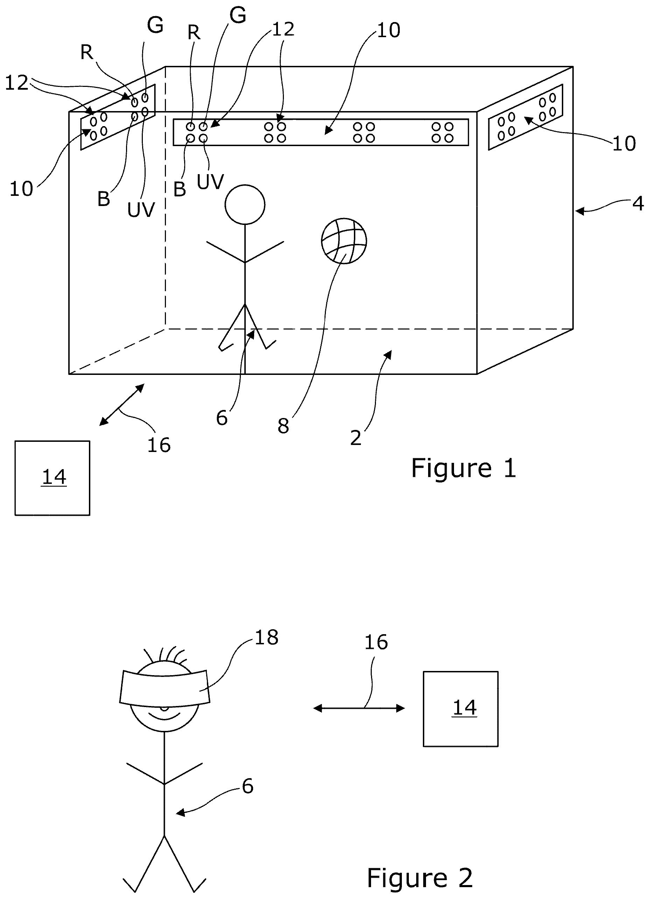

A simplified view of the system according to an embodiment of the present invention is shown in figure 1. An enclosed training area 2 is defined in a housing 4. The training area is of such size, shape and/or form to allow one or more training tasks to be performed with a physical element by a user 6 within the enclosed training area in use. In the illustrated example the physical element is a football 8 and the user 6 is required to undertake a number of tasks relating to trying to kick or save the football once the football moves towards them in use. The reaction time of the user to kick or save the ball and the success rate of the user kicking or saving the ball is recorded. This recording process can be undertaken manually or automatically via one or more sensors and/or the like.

It is noted that the system could be used in a non-enclosed training area but it may be harder to control and/or reproduce the lighting levels required.

The enclosed training area is normally completely dark (i.e. no artificial or natural lighting when the background illumination means are non-operational) and the background illumination within the enclosed training area is controlled by background illumination means in the form of light strips 10. Each light strip contains a plurality of spaced apart light arrays 12. The arrangement and spacing of the light arrays can take any suitable form and/or position so as to provide a relatively uniform background light within the training area when the light arrays are operational in use.

In this particular example, each light array 12 comprises four LEDs or four sets of LED light sources; a red visible light source (R), a blue visible light source (B), a green visible light source (G) and a UV light source (UV); emitting red visible light, blue visible light, green visible light and UV light respectively in use. As explained herein, by adjusting the ratio of the different coloured lights and by adjusting the brightness of the same, different levels and/or combinations of stimulation of the three different cone photoreceptor types and the rod photoreceptors of the user can be obtained.

In one example, the football 8 is provided with physical element illumination means in the form of a fluorescent coating on an external surface thereof, which fluoresces under UV light from the UV light source to provide a visible light that can be seen by the user 6 in use. The degree of luminance emitted from the football can be controlled by controlling the intensity of the UV light source.

In an alternative example, the football can be illuminated by alternative means, such as a light located inside the football or a light in the light array that emits a beam of light that tracks the football in use. In any of the embodiments the degree of luminance generated by the football can be controlled and adjusted as required.

One or more parameters and/or lighting conditions within the training area can be controlled from within the training area (such as for example a display unit on a wall from which various options can be selected by a user) or remotely using a control unit 14 provided remotely to the training area. The control unit 14 can include any or any combination of micro-processing means for emitting and/or processing one or more signals relating to the background illumination means and/or the physical element illumination means; communication means, such as a transmitter and/or receiver for allowing one way or two way communication to take place with the user (as shown by arrow 16), the light arrays, the football and/or the like; memory means for storing software, one or more algorithms, pre-determined lighting options and/or the like.

A simplified view of a further embodiment of the present invention is shown with reference to figure 2. In this arrangement, rather than the user training within an enclosed training area as in figure 1, the user 6 can wear a VR headset 18 that provides a virtual training area and with the lighting arrays 12 built into the VR headset 18. The headset creates an enclosed area around the eyes of the user 6 so that the background illumination comes entirely or substantially entirely from the lighting arrays 12 provided within the VR headset. The cones and rods of the user can still be stimulated in a similar manner to the embodiment in figure 1 but the user does not required a special training area in which to perform their training tasks in. One or more conditions and/or parameters of the system can be controlled through the VR headset and/or can be controlled by a remote control unit as in the embodiment shown in figure 1.

The Applicant's have shown that cone response times of a footballer, who has not been trained on the system of the present invention, reacting to a football coming towards them at relatively high light conditions is up to 50ms faster than for the same event at relatively low light conditions. This means that a football travelling towards a player at 90mph (144.8 km/h) in low light conditions, will travel 10m before the player is able to respond to the football, compared to the ball travelling 8m before the player is able to respond to the football in relatively bright light conditions. Following a pre-determined training period on the system of the present invention, wherein the user repeatedly performs the task of reacting to a football travelling towards them at 90mph (i.e. kicking a football travelling towards them) in low light conditions, the player learns to speed up their response time to the ball. When the player then returns to the same training task at relatively bright light conditions, their reaction time is significantly faster than pre-training levels. The performance advantage gained by the player as a result of training using the system of the present invention has been found to last for days or weeks before retraining is required.

A further more detailed example of the present invention is described below.

### Example 1

A professional goalkeeper's performance was monitored for the entire football season (season 1) directly before they started training on the system of the present invention. The goalkeeper then trained from Feb-May of the same year using the training system of the present invention. The goalkeeper's performance was then monitored for the next entire football season (season 2) directly after they started training on the system of the present invention. The results are shown in table 1.

**Table 1**

| | Football Season 1 (prior to training with system of the present invention) | Pre-invention system training | Post-invention system training | Football season 2 (after training with system of present invention) |
|---|---|---|---|---|
| Games Played | 38 | 28 | 6 | 31 |
| Goals Conceded | 66 | 39 | 3 | 25 |
| Goals conceded per 90 mins | 1.74 | 1.39 | 0.50 | 0.81 |
| Clean Sheets | 10 | 5 | 3 | 8 |
| Clean Sheets % | 26% | 18% | 50% | 26% |

It can be seen from the results in Table 1 that the goalkeepers performance, as measured by the % clean sheet increased from 18% before training on the system of the present invention to 50% after training on the system of the present invention. The number of goals conceded by the goalkeeper fell from 1.39 to 0.50, which is a 64% improvement. It is therefore clear to see that goal keeper training using the system of the present invention for 4 months resulted in a significant improvement in the goal keepers reaction time and performance.

### Example 2

A further professional goalkeeper's performance was monitored in a similar manner as for the first goalkeeper. The results of this further professional goalkeeper's performance are shown in Table 2, with further recorded performance parameters shown.

**Table 2**

| | Football Season 1 (prior to training with system of the present invention) | Pre-invention system training | Football season 2 (after training with system of present invention) |
|---|---|---|---|
| Games Played | 30 | 21 | 34 |
| Shots on Target | 132 | 52 | 108 |
| Saves | 95 | 38 | 86 |
| Save % | 72% | 73% | 80% |
| Clean Sheets | 10 | 8 | 17 |
| Clean Sheets % | 33.3% | 38% | 50% |
| Goals Against | 41 | 27 | 24 |
| Goals against per 90 mins | 1.37 | 1.29 | 0.71 |

The results in Table 2 clearly show an increase in performance after the goalkeeper trained with the system of the present invention. On 17 occasions (out of 34 games) following training on the system of the present invention, the goalkeeper saved all shots (100%). The number of goals conceded dropped significantly after training with the system of the present invention (from 1.29 to 0.71). This demonstrates a significant increase in the performance of the goalkeeper following training on the system of the present invention.

### Example 3

It will be appreciated that the cost of rehabilitating football players after injury is a significant cost for professional football clubs to incur and with the system of the present invention, players can quickly be brought back to full fitness by training on the system without exposure to the injury risks of playing actual matches or in group training. An example of the performance return of a player following injury is shown in Table 3 below.

The rehabilitation of the player was split into three phases; phase 1 included solo training to activate the muscles and brain using the system of the present invention; phase 2 including contextual training drills using the system of the present invention; and phase 3 return to match fitness.

**Table 3**

| | Before Injury with no training on the system of the present invention | After Injury following training on system of the present invention |
|---|---|---|
| Passes | 35.1 | 40.8 |
| Pass Accuracy | 80% | 86% |
| Through passes | 0.4 | 0.8 |
| Through pass accuracy | 32% | 40% |
| Second Assists | 0.0 | 0.3 |
| Passes to Penalty Area | 1.7 | 2.2 |
| Accuracy of passes to penalty area | 45% | 54% |

From the Table 3, it can be seen that following player rehabilitation using the system of the present invention, they improved their pass rate by 12.5%, their pass accuracy increased by 6%. The player doubled their through passes and their accuracy improved by 20%. The number of accurate passes into the penalty area increased by 16.67%. This supports the beneficial effects of the present invention.

### Example 4

A yet further professional goalkeeper's performance was monitored in a similar manner as for the first and second goalkeepers. The results of this yet further professional goalkeeper's performance are shown in Table 4.

**Table 4**

| | Before training on the system of the present invention | After training on system of the present invention |
|---|---|---|
| % saves | 66.7% | 80.56% |
| Goals conceded | 1.5084 | 0.681 |
| Shots faced | 81 | 36 |
| Saves | 54 | 29 |
| Games Played | 19 | 12 |
| Clean Sheets | 4 | 7 |
| % Clean Sheets | 21.05% | 58.33% |

The % of saves made by the goalkeeper after training with the system of the present invention increased by 17.20% and the % of goals conceded decreased by 57%. The % clean sheet improvement was 63%. These results further support the beneficial effect of the system of the present invention.

### Theory behind light adjustment benefits of the system of the present invention

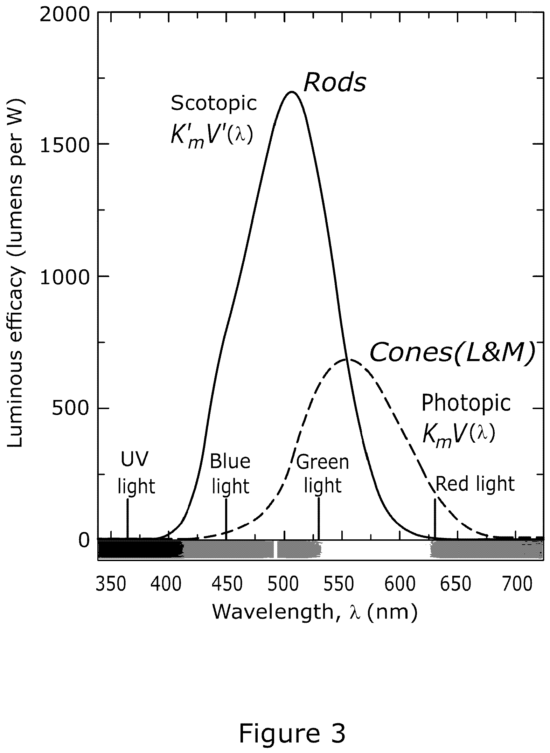

Although the lighting levels of the background illumination and/or the training element illumination can be arbitrarily selected by a user of the system by selecting one of a plurality of possible illumination conditions, the background illumination and/or the training element illumination can also be optimized by a user selecting one of a number of pre-determined lighting/training conditions provided in the system. In order to demonstrate one way in which the Applicants have optimized the plurality of pre-determined lighting/ training conditions provided by the system, they have considered how light levels effects stimulation of the cones and rods in a human eye. As shown in figure 3, the L-, M- and S- cones and rods all have different spectral sensitives, and so are affected differently by different wavelengths of light. The scotopic (black curve) and photopic (white curve) luminous efficacy functions are shown in lumens per W (lm/W) in figure 3.

V'(λ) and V(λ) are the scotopic and luminance efficiency functions respectively, which are normalized to peak and one. K'ₘ =1700 lm/W and Kₘ=683 lm/W are the maximum luminance efficacies, which occur at 507nm and 555nm for the scotopic and photopic functions respectively. By multiplying K'ₘ by V'(λ) and Kₘ by V(λ), the efficacy functions shown in the Figure 3 can be obtained. It is to be noted that the photopic function reflects mainly the sensitivities of the L- and M-cones with little or no input from the S-cones. The short vertical lines represent the wavelengths of light that can be provided by the illumination means in the system of the present invention in one embodiment, which in this example are UV light, blue light, green light and red light. Other combinations of lights for use in the present invention are also envisaged.

The scotopic range refers to the range in which light affects only the rods, and the photopic range refers to the range in which light only affects the cones. The higher the efficacy in lumens per watt (lm/W), the greater the effect of light of a given wavelength on either the rods (scotopic lm/W) or the cones (photopic lm/W). Thus, the UV light used in the system in one embodiment is found to have very little direct effect on the rods or cones of a human eye. The blue light has more effect on the rods than cones and the red light has more effect on cones than rods. By varying the intensities of the lights within the system of the present invention, the Applicant can alter the relative excitation of the rods and cones in the human eye.

Two points are worth noting. Firstly, if scotopic levels are high, the rods will saturate and cease to contribute to vision. Secondly, photopic measurements of illumination largely ignore the effects of the light on the S cones.

UV light, which is also known as black light because it is nearly invisible to a human eye, can be used in one embodiment as a source of background illumination in the system of the present invention. As can be seen in Figure 3, UV light has very low scotopic and photopic luminous efficacies and so will not significantly light adapt (and thus speed up) either the rods or the cones. Hence, in one embodiment of the present invention, the purpose of the UV light is to excite a fluorescent coating on the physical training element, such as for example a football or pitch markings, causing them to emit visible light in a different part of the spectrum. For example, the fluorescent football can appear orange and the pitch markings can appear orange or a violet-white.

Since the ball is orange, its trajectory and shape will be detected mainly by L-cones and M-cones, but not by the S-cones. This is appropriate because the L- and M-cones feed into the visual pathways with which the human eye sees motion and detail best (known as the "magnocellular" and parvocellular" pathways, respectively), there being little or no input into these pathways from the S-cones.

The dependence of tasks involving motion and detail primarily on L- and M-cones is one of the reasons why the photopic V(λ) function is the sum of the L- and M-cone spectral sensitivities with no S-cone contribution.

When the background illumination is low and the visual system has time to adapt to the darkness, the rods are likely to be sensitive enough to detect the fluorescent orange ball along with the cones. However, the rod generated image of the football may be substantially more delayed than the cone generated image and, moreover, will be of poor spatial resolution. This potentially unwanted rod contribution, which will blur the image seen by a user in time and space, can be reduced in the system of the present invention by illuminating the training area via the background illumination means with a blue light source. This light excites rods much more than the cones, such that adding a dim blue light can reduce rod sensitivity without affecting the L- or M- cones. In one example of the present invention, players reported that their training seemed more effective when the background illumination levels were based on a low level blue light.

### Examples of the some of the pre-determined positions and ratios that the background illumination means and/or physical element illumination means can be adjusted to in the system of the present invention in one embodiment (measured in photopic lux).

Okkulo Light Levels (Level 4) (without red (R), blue (B), green (G) 10%)
Photopic - Cones
Goalkeeper area illumination - 17 milli lux right side (RS) and left side (LS) 12 milli lux
Outfield player illumination - 12.9 milli lux - 14 milli lux
Scotopic - Rods
Goalkeeper area illumination- 83.8 milli lux (RS) and 57.7 milli lux (LS) 29.5 milli-lux
Outfield player illumination - 100.5 milli lux
Okkulo Light Levels (Level 4) (with RBG 10% (i.e. the red, blue, green light sources set to 10% of their capacity)
Photopic - Cones
Goalkeeper area illumination - 62.6 milli lux (RS) 46.9 milli lux (LS) 31.9 milli lux
Outfielder illumination - 68.9 milli lux
Scotopic - Rods
Goalkeeper area illumination 390.5 milieux (RS) 166.7 milli lux (LS) 268.1 milli lux
Outfielder player illumination - 485.4 milli lux
Photopic - Cones
Player view Goalkeeper illumination - 32.8 milli lux
Player view Outfielder illumination- 42.6 milli lux
Scotopic - Rods
Player view Goalkeeper illumination - 73.7 milli lux
Player view | Outfielder illumination - 97.6 milli lux
THE BALL SCOTOPIC RODS 175 MILLI LUX
THE BALL PHOTOPIC CONES - 0.41 LUX
BLUE FULL (Level 1) -
Photopic - Cones
Goalkeeper area illumination - 0.455 lux (RS) 197.1 milli lux (LS) 160.8 milli lux
Outfielder Player illumination - 0.468 lux
Scotopic - Rods
Goalkeeper area illumination 15.12 lux (RS) 9.16 lux (LS) 7.63 lux
Outfielder player illumination- 18.47 lux
Photopic - Cones
Player view Goalkeeper illumination- 41.6 milli lux
Player view Outfielder illumination- 49.2 milli lux
Scotopic - Rods
Player view Goalkeeper illumination 400.2 milli lux
Player view Outfielder illumination - 529.7 milli lux
THE BALL SCOTOPIC RODS - 769.1 MILLI LUX
THE BALL PHOTOPIC CONES - 0.980 LUX
RED FULL (Level 3)
Photopic - Cones
Goalkeeper area illumination 1.864 lux (RS) 0.871 lux (LS) 0.786 lux
Outfielder Player illumination - 2.138 lux
Scotopic - Rods
Goalkeeper area illumination - 133.4 milli lux (RS) 86.5 milli lux (LS) 70.8 milli lux
Outfielder player illumination - 180.3 milli lux
Photopic - Cones
Player view Goalkeeper illumination- 97.6 milli lux
Player view Outfielder illumination - 80.3 milli lux
Scotopic - Rods
Player view Goalkeeper illumination- 88.0 milli lux
Player view Outfielder illumination- 86.9 milli lux
THE BALL SCOTOPIC RODS - 158.1 milli lux
THE BALL PHOTOPIC CONES - 0.703 LUX
GREEN FULL (Level 2)
Photopic - Cones
Goalkeeper area illumination 5.001 lux (RS) 2.25 lux (LS) 2.857 lux
Outfielder Player illumination - 15.61 ux
Scotopic - Rods
Goalkeeper area illumination 9.54 milli lux (RS) 5.64 lux (LS) 4.66 lux
Outfielder player illumination 13.99 lux
Photopic - Cones
Player view Goalkeeper illumination - 215.3 milli lux
Player view Outfielder illumination- 232.9 milli lux
Scotopic - Rods
Player view Goalkeeper illumination- 456.7 milli lux
Player view Outfielder illumination- 649.9 milli lux
THE BALL SCOTOPIC RODS - 786.5 milli lux
THE BALL PHOTOPIC CONES - 0.745 LUX
NORMAL - Standard Level
Photopic - Cones
Goalkeeper area illumination 0.477 K lux (RS) 163.21 lux (LS) 85.3 lux
Outfielder Player illumination 0.399 K Lux
Scotopic - Rods
Goalkeeper area illumination 0.903 K lux (RS) 361.7 lux (LS) 188.3 lux
Outfielder player illumination 0.903 K lux
Photopic - Cones
Player view Goalkeeper illumination- 12.30 lux
Player view Outfielder illumination- 19.85 lux
Scotopic - Rods
Player view Goalkeeper illumination- 29.58 lux
Player view Outfielder illumination - 38.68 lux
THE BALL SCOTOPIC RODS - 64.48 lux
THE BALL PHOTOPIC CONES - 92.1 LUX

### Examples of the system of the present invention providing a therapeutic effect

In one test, a patient suffering from Attention Deficit Hyperactivity Disorder volunteered to undertake a plurality of training tasks using the system of the present invention for a number of weeks, wherein the background illumination of the system was set predominantly to low level blue lighting. After a few weeks of training using the system of the present invention, the focus of the patient when performing the same training tasks, but also other everyday tasks, outside of the system improved significantly. As such, there is evidence to support the system of the present invention being used for disorders and/or conditions where users have difficulty in focusing or concentrating.

In another test, a patient who had experienced a brain trauma and was struggling to concentrate and remember things in everyday life volunteered to undertake a plurality of training tasks using the system for a number of weeks, wherein the background illumination of the system was set predominantly to low level blue lighting. After a few weeks of training using the system of the present invention, the patient's concentration levels and memory when performing both the same training tasks and other everyday tasks, outside of the system improved significantly. As such, there is evidence to support the system of the present invention being used for disorders and/or conditions relating to brain trauma, concussion and/or the like where users may be experiencing difficulties in focusing or concentrating.

In a further test, patients how were undergoing rehabilitation after surgery or injury volunteered to undertake a plurality of training tasks using the system for a number of weeks, wherein the background illumination of the system was set predominantly to low level red and/or green lighting. After a few weeks of training using the system of the present invention, the patient's depth perception was found to have significantly improved.

The above examples help to demonstrate that the system of the present invention could offer a therapeutic benefit for a number of different conditions, diseases, disorders, in injury rehabilitation and/or the like.

It will be appreciated by persons skilled in the art that further experimentation using different combinations of lighting intensities, wavelengths and/or the like according to the system of the present invention could provide optimized conditions for a much wider range of training, rehabilitation and/or therapeutic benefits.

Referring to Figure 4, if three primary light sources, red (R), green (G) and blue (B) light sources are used for the background illumination means in the system of the present invention according to one embodiment, and are mixed together in different ratios, a resulting background illumination colour that falls within the white drawn triangle on figure 4 can be realised (the white drawn triangle is created by joining up the R, G, B plots on the chromaticity diagram). It can be seen from this diagram this other colour combinations could be used to provide suitable background illumination. Furthermore, if four primary colours were used, a four sided boundary would be created that would enclose more possible background colour options than the illustrated white triangle.

Thus, it can be seen from figure 4, that by adding and/or mixing different coloured light sources together in the system at different intensities and/or ratios a large range of background illumination possibilities can be produced. Each illumination possibility can create optimized illumination for achieving a particular beneficial effect in a user.

It can also be seen from figure 4, that two different light sources can be used to provide the background illumination. For example, If C1 (blue) and C2 (orange) were the two different light sources and a dotted line is drawn between the same on figure 4, and different colours can be produced that fall along this dotted line that can provide the background illumination.

### Example 5

### Methodology

A further study was undertaken to investigate whether the use of the system of the present invention improves the visual abilities of athletes. The study recruited 24 elite soccer athletes aged 18-30 years and any gender who did not have pre-existing visual impairments. The athletes were divided into an experimental group and a control group using a randomised controlled trial design. Each group were monitored for their visual abilities at the start and end of a six week training period. The experimental group performed a period of visual soccer training drills using the system of the present invention in the six weeks between the pre and post visual ability tests, whereas the control group performed the same visual soccer training drills without using the system of the present invention.

The vision ability test protocol used for the pre and post training period tests measured eight different metrics, which all have different functions in the visual system, especially during sport. They are:
- Visual acuity in movement: the ability to see objects while in motion;
- Recognition time: the time it takes to identify an object;
- Stereopsis: the ability to perceive depth;
- Sensorial reaction time: the time it takes to respond to a sensory stimulus (eye to brain);
- Motor reaction time: the time it takes to respond through movement (brain to muscle);
- Identification speed: the speed at which objects are identified;
- Contrast sensitivity: the ability to distinguish between objects with low contrast;
- Anticipation: the ability to predict the movement of objects.

The experimental group performed a minimum of two 30 minute supervised training sessions per week using the system of the present invention during the six week training period. The control group performed the same minimum two 30 minute supervised sessions per week but without using the system of the present invention during the six week training period. There was no maximum amount of time the experimental group or control group athletes could train for during the training period. Throughout the six week training period, the light levels which the athletes trained under for the experimental changed as follows:
Week 1 - Blue light only
Week 2 - Green light only
Week 3 - Red light only
Week 4 - UV light with a 10% mix of Red, blue and green light (hereinafter referred to as Okkulo Light).
Weeks 5 & 6 - Red and Okkulo light only.

The supervised training included the athletes performing simple groups of ball drills designed to make full use of a pre-defined enclosed area/pitch on an individual basis with a coach. A ball machine was used for at least part of the sessions so ball speed could remain constant (50mph). The drills involved ball control, dribbling and shooting.

### Results

Using statistical analysis (Wilcoxon test) the following results were obtained (V = part of the test statistic relating to the sum of ranks with a positive sign; p = probability; n= number of athletes):
1. Visual acuity (the athlete's ability to distinguish shapes and the details of objects while they are moving):
   Experimental group V=66; p=0.0019; n=11
   Control group V= 10.5; p=0.93; n=11

The experimental group showed a 10% increase in visual acuity post-test period compared to the control group. This test reflects the athlete's ability to see in better detail, the players, the ball and all on-field action, when in motion

### 2. Recognition Time (how well an athlete can correctly process images visually and assess what to do with the information):

Experimental group V=3.5; p=0.0049; n=11
Control group V=15; p=0.98; n=11

The experimental group showed a 56% increase in recognition time post-test period compared to the control group. This test reflects the ability of an athlete to see, process and act on information presented on pitch significantly quicker, allowing for a higher percentage of successful on-field actions.

### 3. Identification Test (peripheral vision test to test the athlete's ability to use their peripheral vision currently and successfully):

Experimental group V=55; p=0.0027, n=11
Control group V=22, p=0.094, n=11

The experimental group showed a 10% increase in peripheral vision abilities post-test period compared to the control group. This test reflects the athlete's ability to process information out of the clear line of sight, allowing for greater accuracy on in-game actions.

### 4. Advanced Anticipation Time (the amount of time which an athlete judges an object ahead of its actual position):

Experimental group V=2; p=0.0059; n=10
Control group V=3; p=0.31, n=8

The experimental group showed a 60% increase in anticipation time post-test period compared to the control group. This test reflects the athletes ability ot predict where the ball will be during its flight path, allowing for better contact, touching, catching or saving of the ball.

### 5. Stereopsis (the visual ability to perceive the world in three dimensions, which then allows a person to judge where an object is relative to him/her):

Experimental group V=6; p=0.015; n=11
Control group V=21; p=0.69; n=11

The experimental group showed a 20% increase in stereopsis post-test period compared to the control group. Improved stereopsis means the athlete can perceive depth much better, thereby allowing the athletes to judge ball flight and movement better.

### 6. Reaction Time (sensorial) (the speed in which an athlete processes change from eye to brain):

Experimental group V=0; p=0.0019; n=11
Control group V=17.5; p=0.091, n=11

The experimental group showed a 16% increase in sensorial reaction time post-test period compared to the control group. This test reflects the ability of the athlete to process and make split second decisions between eye to brain.

Thus, the above results show that a significant improvement of athletes visual abilities was found after using the system of the present invention for a six week period.

## Claims

1. A training system, said training system including:
- background illumination means (10) to provide background illumination for a user's vision in use;
- at least one physical element (8) relating to a training task to be performed by the user in use;- wherein physical element illumination means are provided on, in and/or associated with the at least one physical element to allow the physical element to be illuminated in use;
- wherein the background illumination means includes an Ultra Violet (UV) light source and the physical element illumination means includes a UV sensitive coating that emits visible light when UV light from the UV light source is directed onto the same in use;
**characterized in that**:
adjustment means are provided to allow adjustment of the background illumination generated by at least the UV light source of the background illumination means, thereby allowing adjustment of the visible light generated by the physical element illumination means in use.

2. The training system according to claim 1, wherein the background illumination provided by the background illumination means also includes visible light and the adjustment means allows adjustment of the visible light.

3. The training system according to any preceding claim, wherein the adjustment means are arranged to allow adjustment of one or more parameters and/or conditions relating to the background illumination, the physical element illumination, the background illumination means and/or the physical element illumination means; to adjust the difficulty of the training task being performed by a user of the system; to adjust the stimulation of one or more cones and/or rods of a human eye of the user; to adjust the stimulation of a user's vision in the mesopic, photopic and/or scotopic range(s); and/or arranged to adjustment of any or any combination of: wavelength, colour and/or composition of the light emitted from the illumination means, the brightness of the light emitted from the illumination means, the intensity of the light emitted from the illumination means, the type of light emitted from the illumination means, the ratio of two or more different lights of different wavelengths emitted by the illumination means, or the contrast between the light emitted by the background illumination means and the physical element illumination means.

4. The training system according to any preceding claim, wherein the adjustment means are arranged to allow adjustment of two or more parameters and/or conditions relating to the background illumination, the background illumination means, the physical element illumination and/or the physical element illumination means simultaneously and/or independently of each other.

5. The training system according to any preceding claim, wherein the system includes a control unit (14) or device and the adjustment means are provided on and/or associated with the control unit or device, and optionally the control unit or device includes hardware and/or software for carrying out one or more processes, memory means for storing data relating to the system; and/or communication means for communicating data with and/or between one or more electronic devices.

6. The training system according to any preceding claim, wherein the light emitted from the background illumination means and/or physical element illumination means is polarised light, non-polarised light, partially polarised light, and/or different states of polarised light; is a continuous or substantially continuous light; a non-continuous or substantially non-continuous light; a pulsed light; and/or a predetermined or random pattern, sequence or sequences of light.

7. The training system according to any preceding claim, wherein the background illumination means includes a first illumination means capable of emitting a first colour and/or wavelength of light, and at least a second illumination means capable of emitting at least a second colour and/or wavelength of light, the first and second light colours and/or wavelengths being different; or wherein the background illumination emits red, blue and/or green visible light

8. The training system according to any preceding claim, wherein the UV light is UVA light.

9. The training system according to any preceding claim, wherein filter means are provided on and/or associated with the background illumination means and/or physical element illumination means to provide illumination of one or more colours, wavelengths and/or patterns.

10. The training system according to any preceding claim, wherein the background illumination provided by the background illumination means is adjustable from 0 to 1000cd/m² and/or the illumination provided by the physical element illumination means is adjustable to a level equal to or greater than 0.001cd/m².

11. The training system according to any preceding claim, wherein the physical element illumination means are locatable directly in or on the physical element.

12. The training system according to any preceding claim, wherein the system includes a training area (2) provided in an enclosed space or area, and optionally a radiation absorbing coating and/or layer is provided on and/or associated with an interior surface of the enclosed training area or space.

13. The training system according to any of claims 1-11, wherein the system includes an item of eyewear and/or headwear for wearing by a user and, when worn, the eyewear or headwear provides an enclosed or substantially enclosed space around the user's eye or eyes, with the background illumination means arranged to provide background illumination within the enclosed or substantially enclosed space around the user's eye or eyes.

14. The training system according to any preceding claim, wherein sensing means are provided for sensing the position, speed and/or movement of one or more of the physical elements in use.

15. A method of using a training system, said method including the steps of
- providing background illumination for a user of the system's vision using background illumination means;
- providing at least one physical element relating to a training task and performing the training task using the physical element;
- illuminating the physical element using physical element illumination means provided on, in and/or associated with the at least one physical element;
- wherein the background illumination means includes an Ultra Violet (UV) light source and the physical element illumination means includes a UV sensitive coating that emits visible light when UV light from the UV light source is directed onto the same in use;
**characterized in that** the method further includes the step of:
adjusting the background illumination generated by at least the UV light source of the background illumination means, thereby allowing adjustment of the visible light generated by the physical element illumination means using adjustment means.

## Patentansprüche

1. Trainingsystem, wobei das genannte Trainingsystem Folgendes beinhaltet:
- Hintergrundbeleuchtungsmittel (10) zum Bereitstellen von Hintergrundbeleuchtung für das Sehen eines Benutzers bei der Verwendung;
- mindestens ein physisches Element (8), das sich auf eine bei Verwendung vom Benutzer auszuführende Trainingsaufgabe bezieht;
- wobei an, in und/oder in Verbindung mit dem mindestens einen physischen Element Beleuchtungsmittel für das physische Element bereitgestellt sind, damit das physische Element bei Verwendung beleuchtet werden kann;
- wobei das Hintergrundbeleuchtungsmittel eine Ultraviolett- (UV) -Lichtquelle beinhaltet und das Beleuchtungsmittel für das physische Element eine UV-empfindliche Beschichtung beinhaltet, die sichtbares Licht abgibt, wenn UV-Licht von der UV-Lichtquelle bei Verwendung auf dasselbe gerichtet wird;
**dadurch gekennzeichnet, dass**:
Anpassmittel vorgesehen sind, um das Anpassen der durch zumindest die UV-Lichtquelle des Hintergrundbeleuchtungsmittels erzeugten Hintergrundbeleuchtung zu ermöglichen, wodurch bei Verwendung die Anpassung des durch das Beleuchtungsmittel für das physische Element erzeugten sichtbaren Lichts möglich ist.

2. Trainingssystem nach Anspruch 1, wobei die durch das Hintergrundbeleuchtungsmittel bereitgestellte Hintergrundbeleuchtung auch sichtbares Licht beinhaltet und das Anpassmittel die Anpassung des sichtbaren Lichts ermöglicht.

3. Trainingssystem nach einem der vorhergehenden Ansprüche, wobei die Anpassmittel angeordnet sind zum Ermöglichen der Anpassung von einem bzw. einer oder mehreren Parametern und/oder Bedingungen in Bezug auf die Hintergrundbeleuchtung, die Beleuchtung des physischen Elements, das Hintergrundbeleuchtungsmittel und/oder das Beleuchtungsmittel für das physische Element; zum Anpassen der Schwierigkeit der von einem Benutzer des Systems ausgeführten Trainingsaufgabe; zum Anpassen der Stimulation eines oder mehrerer Zapfen und/oder Stäbchen eines menschlichen Auges des Benutzers; zum Anpassen der Stimulation des Sehvermögens eines Benutzers im mesopischen, photopischen und/oder skotopischen Bereich; und/oder zur Anpassung von jedem oder jeder Kombination der folgenden angeordnet sind: Wellenlänge, Farbe und/oder Zusammensetzung des von den Beleuchtungsmitteln abgegebenen Lichts, der Helligkeit des von den Beleuchtungsmitteln abgegebenen Lichts, der Intensität des von den Beleuchtungsmitteln abgegebenen Lichts, der Art des von den Beleuchtungsmitteln abgegebenen Lichts, des Verhältnisses von zwei oder mehr verschiedenen, von den Beleuchtungsmitteln abgegebenen Lichtern unterschiedlicher Wellenlängen oder des Kontrasts zwischen dem von den Hintergrundbeleuchtungsmitteln und dem von den Beleuchtungsmitteln für das physische Element abgegebenen Licht.

4. Trainingssystem nach einem der vorhergehenden Ansprüche, wobei die Anpassmittel zum Ermöglichen der Anpassung von zwei oder mehr Parametern und/oder Bedingungen in Bezug auf die Hintergrundbeleuchtung, das Hintergrundbeleuchtungsmittel, die Beleuchtung des physischen Elements und/oder Beleuchtungsmittel für das physische Element gleichzeitig und/oder unabhängig voneinander angeordnet sind.

5. Trainingssystem nach einem der vorhergehenden Ansprüche, wobei das System eine Steuereinheit (14) oder -vorrichtung beinhaltet und die Anpassmittel an der Steuereinheit oder -vorrichtung bereitgestellt sind und/oder derselben zugeordnet sind und, wahlweise, die Steuereinheit oder -vorrichtung Hardware und/oder Software zum Durchführen von einem oder mehreren Prozessen, Speichermittel zum Speichern von Daten in Bezug auf das System; und/oder Kommunikationsmittel zum Austauschen von Daten mit und/oder zwischen einem oder mehreren elektronischen Vorrichtungen beinhaltet.

6. Trainingssystem nach einem der vorhergehenden Ansprüche, wobei das von dem Hintergrundbeleuchtungsmittel und/oder Beleuchtungsmittel für das physische Element abgegebene polarisiertes Licht unpolarisiertes Licht, teilweise polarisiertes Licht und/oder verschiedene Zustände von polarisiertem Licht ist; ein kontinuierliches oder im Wesentlichen kontinuierliches Licht; ein nicht-kontinuierliches oder im Wesentlichen nicht-kontinuierliches Licht; ein gepulstes Licht; und/oder ein vorbestimmtes oder zufälliges Muster, eine Lichtfolge oder -folgen ist.

7. Trainingssystem nach einem der vorhergehenden Ansprüche, wobei das Hintergrundbeleuchtungsmittel ein erstes Beleuchtungsmittel, das eine erste Farbe und/oder Wellenlänge von Licht abgeben kann, und mindestens ein zweites Beleuchtungsmittel, das mindestens eine zweite Farbe und/oder Wellenlänge von Licht abgeben kann, beinhaltet, wobei die erste und die zweite Lichtfarbe und/oder Wellenlänge verschieden sind; oder wobei die Hintergrundbeleuchtung rotes, blaues und/oder grünes sichtbares Licht abgibt.

8. Trainingssystem nach einem der vorhergehenden Ansprüche, wobei das UV-Licht UVA-Licht ist.

9. Trainingssystem nach einem der vorhergehenden Ansprüche, wobei an dem Hintergrundbeleuchtungsmittel und/oder dem Beleuchtungsmittel für das physische Element Filtermittel bereitgestellt sind oder denselben zugeordnet sind, um Beleuchtung mit einer oder mehreren Farben, Wellenlängen und/oder Mustern bereitzustellen.

10. Trainingssystem nach einem der vorhergehenden Ansprüche, wobei die von dem Hintergrundbeleuchtungsmittel bereitgestellte Hintergrundbeleuchtung von 0 bis 1000 cd/m² einstellbar ist und/oder die von dem Beleuchtungsmittel für das physische Element bereitgestellte Beleuchtung auf eine Höhe von größer oder gleich 0,001 cd/m² einstellbar ist.

11. Trainingssystem nach einem der vorhergehenden Ansprüche, wobei die Beleuchtungsmittel für das physische Element direkt in oder an dem physischen Element positionierbar sind.

12. Trainingssystem nach einem der vorhergehenden Ansprüche, wobei das System einen in einem geschlossenen Raum oder Bereich bereitgestellten Trainingsbereich (2) beinhaltet und, wahlweise, eine strahlungsabsorbierende Beschichtung und/oder Schicht auf einer Innenfläche des geschlossenen Trainingsbereichs oder -raums bereitgestellt und/oder mit derselben verbunden ist.

13. Trainingssystem nach einem der Ansprüche 1 bis 11, wobei das System ein vor den Augen und/oder einen auf dem Kopf zu tragenden Artikel zum Tragen durch einen Benutzer beinhaltet und, wobei der vor dem Auge oder den Augen oder auf dem Kopf zu tragende Artikel, wenn er getragen wird, einen geschlossenen oder im Wesentlichen geschlossenen Raum um das Auge oder die Augen des Benutzers bildet, wobei das Hintergrundbeleuchtungsmittel zum Bereitstellen von Hintergrundbeleuchtung innerhalb des geschlossenen oder im Wesentlichen geschlossenen Raums um das Auge oder die Augen des Benutzers angeordnet sind.

14. Trainingssystem nach einem der vorhergehenden Ansprüche, wobei Erfassungsmittel zum Erfassen der Position, Geschwindigkeit und/oder Bewegung eines oder mehrerer der verwendeten physikalischen Elemente bereitgestellt sind.

15. Verfahren zur Verwendung eines Trainingssystems, wobei das genannte Verfahren die folgenden Schritte beinhaltet:
- Bereitstellen von Hintergrundbeleuchtung für das Sehen eines Benutzers des Systems bei Verwendung von Hintergrundbeleuchtungsmitteln;
- Bereitstellen von mindestens einem physischen Element, das sich auf eine Trainingsaufgabe bezieht, und Ausführen der Trainingsaufgabe unter Verwendung des physischen Elements;
- Beleuchten des physischen Elements unter Verwendung von Beleuchtungsmitteln für das physische Element, die an, in und/oder in Verbindung mit dem mindestens einen physischen Element bereitgestellt sind;
- wobei das Hintergrundbeleuchtungsmittel eine Ultraviolett- (UV) -Lichtquelle beinhaltet und das Beleuchtungsmittel für das physische Element eine UV-empfindliche Beschichtung beinhaltet, die sichtbares Licht abgibt, wenn UV-Licht von der UV-Lichtquelle im Gebrauch auf dasselbe gerichtet wird;
**dadurch gekennzeichnet, dass** das Verfahren ferner den folgenden Schritt beinhaltet:
Anpassen der durch zumindest die UV-Lichtquelle des Hintergrundbeleuchtungsmittels erzeugten Hintergrundbeleuchtung, wodurch die Anpassung des durch das Beleuchtungsmittel für das physische Element erzeugten sichtbaren Lichts unter Verwendung von Anpassmitteln möglich ist.

## Revendications

1. Système d'entraînement, ledit système d'entraînement incluant :
- un moyen d'éclairage d'arrière-plan (10) destiné à fournir un éclairage d'arrière-plan pour la vision d'un utilisateur, en utilisation ;
- au moins un élément physique (8) relatif à une tâche d'entraînement devant être effectuée par l'utilisateur, en utilisation ;
- dans lequel des moyens d'éclairage d'élément physique sont fournis sur, dans et/ou associés à l'au moins un élément physique pour permettre à l'élément physique d'être éclairé, en utilisation ;
- dans lequel le moyen d'éclairage d'arrière-plan inclut une source de lumière ultraviolette (UV) et le moyen d'éclairage d'élément physique inclut un revêtement sensible aux UV qui émet une lumière visible lorsque la lumière UV provenant de la source de lumière UV est dirigée sur celui-ci, en utilisation ;
**caractérisé en ce que** :
des moyens d'ajustement sont prévus afin de permettre un ajustement de l'éclairage d'arrière-plan généré par au moins cette source de lumière UV du moyen d'éclairage d'arrière-plan, permettant ainsi un ajustement de la lumière visible générée par les moyens d'éclairage d'élément physique, en utilisation.

2. Système d'entraînement selon la revendication 1, dans lequel l'éclairage d'arrière-plan fourni par le moyen d'éclairage d'arrière-plan inclut également la lumière visible et le moyen d'ajustement permet un ajustement de la lumière visible.

3. Système d'entraînement selon n'importe quelle revendication précédente, dans lequel les moyens d'ajustement sont agencés de façon à permettre un ajustement d'un ou de plusieurs paramètres et/ou de conditions relatives à l'éclairage d'arrière-plan, à l'éclairage d'élément physique, au moyen d'éclairage d'arrière-plan et/ou aux moyens d'éclairage d'élément physique ; ajuster la difficulté de la tâche d'entraînement en train d'être effectuée par un utilisateur du système ; ajuster la stimulation d'un ou de plusieurs cônes et/ou bâtonnets d'un œil humain de l'utilisateur ; ajuster la stimulation de la vision d'un utilisateur dans la/les gamme/s mésopiques, photopiques et/ou scotopiques ; et/ou agencés de façon à ajuster n'importe quel élément ou n'importe quelle combinaison d'éléments parmi : longueur d'onde, couleur et/ou composition de la lumière émise à partir du moyen d'éclairage, la brillance de la lumière émise à partir du moyen d'éclairage, l'intensité de la lumière émise à partir du moyen d'éclairage, le type de lumière émis à partir du moyen d'éclairage, le rapport entre deux ou plusieurs lumières différentes ayant des longueurs d'onde différentes émises par le moyen d'éclairage, ou le contraste entre la lumière émise par le moyen d'éclairage d'arrière-plan et les moyens d'éclairage d'élément physique.

4. Système d'entraînement selon n'importe quelle revendication précédente, dans lequel les moyens d'ajustement sont agencés de façon à permettre un ajustement de deux ou de plusieurs paramètres et/ou de conditions relatives à l'éclairage d'arrière-plan, au moyen d'éclairage d'arrière-plan, à l'éclairage d'élément physique et/ou aux moyens d'éclairage d'élément physique, simultanément et/ou indépendamment l'un de l'autre.

5. Système d'entraînement selon n'importe quelle revendication précédente, le système incluant un dispositif ou une unité de commande (14) et les moyens d'ajustement étant prévus sur et/ou associés au dispositif ou à l'unité de commande, et facultativement le dispositif ou l'unité de commande incluant du matériel et/ou du logiciel pour réaliser un ou plusieurs processus, des moyens à mémoire pour stocker des données relatives au système ; et/ou des moyens de communication pour la communication de données avec et/ou entre un ou plusieurs dispositifs électroniques.

6. Système d'entraînement selon n'importe quelle revendication précédente, dans lequel la lumière émise à partir du moyen d'éclairage d'arrière-plan et/ou du moyen d'éclairage d'élément physique est une lumière polarisée, une lumière non polarisée, une lumière partiellement polarisée, et/ou différents états de lumière polarisée ; est une lumière continue ou substantiellement continue ; une lumière non continue ou substantiellement non continue ; une lumière pulsée ; et/ou une séquence ou des séquences de lumière à schéma prédéterminé ou aléatoire.

7. Système d'entraînement selon n'importe quelle revendication précédente, dans lequel le moyen d'éclairage d'arrière-plan inclut un premier moyen d'éclairage apte à émettre une première couleur et/ou longueur d'onde de lumière, et au moins un deuxième moyen d'éclairage apte à émettre au moins une deuxième couleur et/ou longueur d'onde de lumière, les première et deuxième couleurs de lumière et/ou longueurs d'onde étant différentes ; ou dans lequel l'éclairage d'arrière-plan émet une lumière visible rouge, bleue et/ou verte.

8. Système d'entraînement selon n'importe quelle revendication précédente, dans lequel la lumière UV est une lumière UV A.

9. Système d'entraînement selon n'importe quelle revendication précédente, dans lequel des moyens de filtrage sont prévus sur et/ou associés au moyen d'éclairage d'arrière-plan et/ou au moyen d'éclairage d'élément physique pour fournir un éclairage en un/e ou plusieurs couleurs, longueurs d'onde et/ou schémas.

10. Système d'entraînement selon n'importe quelle revendication précédente, dans lequel l'éclairage d'arrière-plan fourni par le moyen d'éclairage d'arrière-plan est ajustable de 0 à 1000 cd/m² et/ou l'éclairage fourni par le moyen d'éclairage d'élément physique est ajustable jusqu'à un niveau égal à ou supérieur à 0,001 cd/m².

11. Système d'entraînement selon n'importe quelle revendication précédente, dans lequel les moyens d'éclairage d'élément physique peuvent être situés directement dans ou sur l'élément physique.

12. Système d'entraînement selon n'importe quelle revendication précédente, le système incluant une zone d'entraînement (2) prévue dans une zone ou un espace fermé/e, et facultativement un revêtement et/ou une couche d'absorption de rayonnement étant prévu/e sur et/ou associé/e à une surface intérieure de la zone ou de l'espace d'entraînement fermé/e.

13. Système d'entraînement selon n'importe lesquelles des revendications 1-11, le système incluant un article de lunetterie et/ou un casque à porter par un utilisateur et, quand il est porté, l'article de lunetterie ou le casque offre un espace fermé ou substantiellement fermé autour de l'œil ou des yeux de l'utilisateur, alors que le moyen d'éclairage d'arrière-plan est agencé de façon à fournir un éclairage d'arrière-plan au sein de l'espace fermé ou substantiellement fermé autour de l'œil ou des yeux de l'utilisateur.

14. Système d'entraînement selon n'importe quelle revendication précédente, dans lequel des moyens de détection sont prévus pour détecter la position, la vitesse et/ou le mouvement d'un ou de plusieurs des éléments physiques en utilisation.

15. Procédé d'utilisation d'un système d'entraînement, ledit procédé incluant les étapes consistant à :
- fournir un éclairage d'arrière-plan pour un utilisateur de la vision du système utilisant un moyen d'éclairage d'arrière-plan ;
- fournir au moins un élément physique relatif à une tâche d'entraînement et effectuer la tâche d'entraînement en utilisant l'élément physique ;
- éclairer l'élément physique en utilisant le moyen d'éclairage d'élément physique fournir prévu sur, dans et/ou associé à l'au moins un élément physique ;
- dans lequel le moyen d'éclairage d'arrière-plan inclut une source de lumière ultraviolette (UV) et le moyen d'éclairage d'élément physique inclut un revêtement sensible aux UV qui émet une lumière visible lorsque la lumière UV provenant de la source de lumière UV est dirigée sur celui-ci, en utilisation ;
**caractérisé en ce que** le procédé inclut en outre l'étape consistant à :
ajuster l'éclairage d'arrière-plan généré par au moins cette source de lumière UV du moyen d'éclairage d'arrière-plan, permettant ainsi un ajustement de la lumière visible générée par les moyens d'éclairage d'élément physique en utilisant les moyens d'ajustement.
